# EUROPEAN PATENT APPLICATION

(11) **EP 3 235 470 A1**
(43) Date of publication of application: **25.10.2017**
(21) Application number: 15869394.5
(22) Date of filing: 15.12.2015
(51) Int. Cl.: A61F 2/02, A61L 27/20, A61L 27/38

(54) **BIOHYBRID FOR THE USE THEREOF IN THE REGENERATION OF NEURAL TRACTS**

(30) Priority: 16.12.2014 ES 201431855
(71) Applicant: Universitat Politècnica de València, 46022 València (ES); Fundación Para La Investigación Biomédica Del Hospital Clínico San Carlos, 28040 Madrid (ES)
(72) Inventor: MONLEÓN PRADAS, Manuel, 46022 València (ES); VALLÉS LLUCH, Ana, 46022 València (ES); MARTÍNEZ RAMOS, Cristina, 46022 València (ES); VILARIÑO FELTRER, Guillermo, 46022 València (ES); BARCIA ALBACAR, Juan Antonio, 28040 Madrid (ES); GÓMEZ PINEDO, Ulises Alfonso, 28040 Madrid (ES)
(74) Representative: Cueto, Sénida
(86) International application number: PCT/ES2015/070909
(87) International publication number: WO 2016/097448

(57) **Abstract**

The invention relates to a biohybrid for the use thereof in the regeneration of neural tracts, comprising an implantable tubular hybrid structure which is degradable and biocompatible and characterised in that it comprises three layers of different porosity: an inner layer a), an intermediate layer b) and an outer layer c), with uninterrupted connection among them, the three layers consisting of the same porous hydrogel based on cross-linked hyaluronic acid, a biohybrid comprising the hybrid tubular structure described, which can contain a fibrous material, preferably poly-L-lactic acid, to a method for producing said tubular hybrid structure and said biohybrid, and to the use of same for regenerating neural tracts in diseases that affect the central nervous system, preferably Parkinson's disease.

## Description

### Field of the invention

The present invention relates to a biohybrid for its use in regenerating neural tracts, that comprises a tubular hybrid scaffold for its use in the regeneration of said tracts, as well as to said tubular scaffold.

### Background of the Invention

Various diseases that affect the central nervous system, such as Parkinson's disease, are currently treated with drugs that relieve symptoms and slow down degeneration. However, it does not exist for many of them a treatment that constitutes a real and effective therapy.

One solution, still in clinical trial, consisting of the cells being grafted *in situ*; however, there is low survival and low effectiveness.

In the peripheral nervous system there are other solutions similar to a duct for nerve regeneration.

The use of hyaluronic acid (HA) in the formation of porous tubes that include other polymers such as poly-L-lactic acid (PLLA) and where Schwann cells are grown and wherein various growth factors, neurotrophic factors, etc are incorporated, is known in the state of the art.

The article "New artificial nerve ducts made with photocrosslinked hyaluronic acid for peripheral nerve 20 regeneration" Sakai Y 1, Matsuyama Y, Takahashi K, Sato T, Hattori T, Nakashima S, Ishiguro N. Biomed Mater Eng. 2007; 17 (3): 191-7, describes tubular porous structures based on photoreticulated HA with a diameter of 1.2 mm and 50 µm pores on which Schwann cells grow. However, the porous tubular structure described in this document differs from the present invention in that it does not consist of three layers. In addition, the composition of the reagent used in the article is a modification of hyaluronic acid with cinnamic acid, and differs from the one of the present invention, which is unmodified hyaluronic acid, which is subsequently cross-linked by reaction with, for example, divinyl sulfone to form an hylan. This difference in composition affects the physicochemical behavior, the rate of degradation and the biological response of the synthesized material, therefore said duct and the one of the present invention are not comparable.

Another article entitled "Electrospun adherent-antiadherent bilayered membranes based on cross-linked hyaluronic for advanced tissue engineering applications" Arnal-Pastor M, Martínez Ramos C., http://www.ncbi.nlm.nih.gov/pubmed?term=Mart%C3%ADnez%20Ramos%20C%5BAu thor%5D&cauthor=true&cauthor_uid=23910318, Pérez Garnés M, Monleón Pradas M, Vallés Lluch A. Mater Sci Eng C Mater Biol Appl. 2013 Oct; 33 (7): 4086-93, refers to a bilayer structure in which one of the layers is HA and the other one is polylactic acid. However, these are not tubular structures and no mention is made of their porosity.

WO2006077085 discloses a biomaterial derived from self-crosslinked HA and neuronal stem cells for the regeneration of damage in the peripheral nervous system and spinal cord. According to claim 5 of this document, the biomaterial may be in the form of tubes with porous walls. However, in the case of WO2006077085, there is not a three-layer structure with different porosity in each of them. This biomaterial is obtained by a) treating the HA derivative with a coating solution, promoting adhesion of neural stem cells, neurite growth and differentiation; b) contacting isolated neural stem cells with the HA derivative of the previous step, and c) culturing and expanding adhered cells in the presence of neurotrophic growth factors selected from beta-FGF (basic fibroblast growth factor), CNTF ciliary neurotrophic factor), BDNF (brain derived neurotrophic factor) and GDNF (glial derived neurotrophic factor) or mixtures thereof.

Patent application US2003060871 relates to a biostable and bioabsorbable tubular structure which may have up to three layers, one of which is always expanded PTFE, and the other two may be polylactic acid and HA. It is a stent for the release of drugs. The different layers may have different pore diameters. It is not said that the HA is nevertheless reticulated. Therefore, it also has essential differences with the tubular scaffold of the present invention.

There is currently no product that allows the transplantation and transportation of neural cells in the brain in a protected way in order to regenerate the nigrostriatal tract in diseases of the central nervous system, and especially Parkinson's disease. This problem can be addressed with the biodegradable and biocompatible tubular scaffold provided by the present invention.

The strategy based on the duct of the present invention overcomes the drawbacks of the state of the art, in such a way that it will allow to repopulate the substantia nigra with dopaminergic cells and protect and guide the process of axonal extension until it reaches the reconnection with the striatum and regenerating in this way the nigrostriatal tract.

The solution proposed by the present invention could also be used for nerve regeneration in the peripheral nervous system.

The solution provided by the present invention has the following advantageous properties:
a) The duct walls admit the free flow of necessary molecules for the development and survival of cells.
b) It protects the transplanted cells in the place that one intends to regenerate and prevents the aggressive cells from reaching its interior.
c) It is biodegradable and completely disappears from the body without the need for new surgical interventions. The speed with which it disappears can also be widely modulated, with small variations in some parameters during the synthesis.
d) Limits or inhibits the effect of the reaction against a foreign body inherent to the grafting of any device in the organism.
e) It can serve as a drug carrier so that these are gradually released in the damaged area.

### DESCRIPTION OF THE INVENTION

Throughout the present specification the terms given below have the following meaning: - "hybrid tubular scaffold", "tubular scaffold", "hybrid tubular duct" and "tubular duct" are used interchangeably.

The present invention relates to a tubular scaffold or hybrid tubular duct of hyaluronic acid, to which poly-L-lactic acid (PLLA) fibers are introduced into the lumen, and can be seeded with cells of interest, such as Schwann cells or glial cells in general and/or neural neurons or precursors *in vitro,* so that the performance thereof can be evaluated.

The tubular structure of the duct should be able to isolate and protect the cells seeded in its lumen from the surrounding external hostile microenvironment, thanks to its microporous morphology, which allows the exchange of oxygen and nutrients and the disposal of waste products. Simultaneously with this exchange task, the microporous structure acts as a barrier to large molecules or cells.

The present invention therefore relates to a degradable and biocompatible implantable tubular scaffold characterized in that it comprises three layers of different porosity: an inner layer a), an intermediate layer b) and an outer layer c), with uninterrupted connection among them and all three composed by the same porous hydrogel based on cross-linked hyaluronic acid.

According to particular embodiments, of the tubular scaffold:
- the inner layer a) has micropores of less than about 1 µm, preferably between about 0.1 and 0.4 µm and more preferably about 0.2 µm,
- the intermediate layer b) has interconnected pores which are larger than the ones of the inner and outer layers, with honeycomb structure, having a size between about 10 and 70 µm, preferably between about 20 and 50 µm, and
- the outer layer, c) has pores smaller than about 12 µm, preferably between about 1 and 12, more preferably between about 1 and 10 µm.

The hybrid tubular duct or tubular scaffold of the invention may have a length of up to 5 cm, as required for the particular application. This duct or hybrid tubular scaffold has a centered inner channel, or lumen, of a diameter between 1 and 0.20 mm. The central channel should be sufficiently wide under wet conditions to allow insertion of PLLA fibers therein.

The present invention also relates to a biohybrid, defined as an assembly comprising a tubular scaffold as the one defined above, plus the product contained therein, which may be for example, cells, it may be a neurotrophin etc., or any combination. Such a biohybrid may harbor Schwann cells or olfactory enveloping glial in its interior, or generally, glial and neural cells.

According to particular embodiments, the biohybrid further comprises growth factors and / or drugs in its lumen. Growth factors are, for example, neurotrophins NGF, BDNF or GDNF. Drugs are, for example, dopaminergics, such as L-dopa.

Preferably the growth factors and / or drugs are found in the lumen embedded in gels or microparticles. The gels could be, for example, injectable and gelifiable peptides *in situ* or solutions of hydrogels such as fibrin, collagen or agarose. The microparticles may have hydrophilic character such as gelatin or hydrophobic like PLLA, or crosslinked gelatin, depending on the character of the molecule to be loaded therein, and varying sizes up to the order of tens of microns.

According to particular embodiments, the biohybrid comprises microfilaments of degradable synthetic polyesters (poly-L-lactic acid, polyglycolic acid (PGA), polycaprolactone or copolymers thereof, for example), nylon or silk up to tens of microns, arranged in parallel in the lumen, which serve as support for adhesion and guidance to cell migration and axon extension.

Furthermore, the present invention also relates to a method for obtaining the defined tubular scaffold characterized by comprising at least:
- arranging a mold for containing said tubular scaffold,
- introducing into said mold a polymer material in the form of fiber (s),
- preparing HA solutions and stirring them in the presence of a cross-linking agent,
- injecting said solutions into the grooves of the mold, obtaining a mold-solutions assembly which cross-links *in situ,*
- freezing the whole mold-solutions obtained, and
- lyophilizing the whole mold-solution thus obtaining microporous HA matrices.

According to particular embodiments the mold is of hydrophobic polymer material, the polymeric material in the form of fibers is hydrophobic polymeric material and the crosslinking agent is divinyl sulfone, glutaraldehyde or carbodiimide.

According to a further specific embodiment of the process, the mold is made of polytetrafluoroethylene, the polymeric material in fiber form is poly-ε-caprolactone and the crosslinking agent is divinyl sulfone, glutaraldehyde or carbodiimide.

The mold used has grooves which can be of various shapes and sizes. For example, they may be grooves of square, circular, oval, irregular section, or any other polygonal shape. The grooves can be up to 3 mm wide.

The fiber-like material used, preferably poly-ε-caprolactone, has a diameter of between about 200 to 2000 µm, preferably between 200 and 1000 µm; and a longer length than the tubular duct.

When divinyl sulfone (DVS) is used as a crosslinking agent, a DVS: HA monomer units ratio of 0.5: 1 or greater may be used.

The HA solutions may be solutions with different concentrations between 0.5% and 8% by weight, always based on weight of HA in 0.2M sodium hydroxide. Preferably the solutions of HA have concentrations of between 1 and 5 % by weight of HA in 0.2 M sodium hydroxide.

The HA solutions are stirred and frozen to -20 °C for a minimum of 5 h. The lyophilization of the mold-solution assembly, for 24 h, is carried out at a pressure below 600 Pa and an initial temperature of about -80 °C. As a product of the lyophilization, microporous matrices of HA are obtained due to the sublimation of water. After the lyophilization step, the tubular scaffolding of the mold, and the rings of the material forming the mold itself are withdrawn, the fibers are withdrawn from the polymer in the form of fibers, obtaining a duct with a centered inner channel, and the HA ducts obtained are hydrated. After the hydration step, PLLA fibers can be inserted in its interior.

The present invention also relates to a tubular scaffold as defined, which is obtained by the process described above.

The present invention also relates to the use of the defined tubular scaffold or of the biohybrid comprising said scaffold, to induce the regeneration of neural tracts and the reconnection of damaged or degenerate neuronal populations.

The present invention also relates to the use of the defined tubular scaffold, or of the biohybrid comprising said scaffold, for their use in regenerating the nigrostriatal tract in diseases affecting the central nervous system, preferably Parkinson's disease and spinal cord injuries.

Millimetric HA ducts have been developed with such a unique porosity of the wall, depth dependent, that allows the diffusion of nutrients or molecular signals, while preventing the cells from penetrating them. The different steps of the manufacturing process of the materials confer them a dimensional and structural stability, and appreciably restrict its swelling in the physiological environment. The tubular ducts have a lumen in which the cells of interest can be seeded in a protected environment. A fiber bundle may be previously included along the lumen, as it was described for synthetic polyesters, nylon or silk, for example of PLLA, to facilitate cell migration or cell growth. These ducts have shown to be compatible with Schwann cells (SCs), as demonstrated in *in vitro* cultures. The especially custom-made 3-dimensional hydrogel represents a favorable environment for cells in terms of their viability, migration and distribution, since they proliferate in the same order as they do on other substrates more appropriate for cells, such as PLLA. In spite of the typical characteristic of low adhesion of HA that usually prevents a good cellular proliferation, SC cells can cover the lumen from one end to another, of ducts of several millimeters, thus forming a continuous layer based on cell-cell junctions. In addition, cultured glial cells within the ducts produce significant amounts of structural myelin proteins over time, even in the absence of axons. For all these reasons, new porous HA ducts are a promising strategy for the restoration of damaged neuronal tracts.

The duct that has been developed is a bridge of superior potential for the regeneration of nervous tracts for several reasons:
First, a special feature of the duct is the triple layer porous wall, which results in a more controlled pore distribution than other comparable devices.

The unique surfaces of the duct wall are capable of preventing the migration of grafted cells out of the channel, forming a barrier to astrocytes, macrophages and other host cells to prevent them from interacting physically with the interior.

In spite of this, microporosity dimensions allow the flow of different molecules such as nutrients, waste products, diluted gases and cytokines and other molecular signals involved in cellular communication and regulation. Thus, the wall compartments allow the exchange of nutrients and cellular debris, and avoid contact between the grafted cells and the host cells. As degradation occurs and the internal porous structure of large pores is exposed, the wall substrate should mimic the surrounding tissue in terms of pore shape and size, similar to those of brain tissue.

Secondly, the duct substrate consists of chains of hyaluronic acid, a component of the extracellular matrix, with a low immune response to the host, which should be ideal for grafting purposes, while being biodegradable and biocompatible. In addition, although cross-linked hyaluronic acid is a highly hygroscopic gel, the lyophilization process limits dimensional variation due to swelling in aqueous solutions. This is a key factor to take into account in order to consider its surgical implantation, since the nerves and soft tissues in the central nervous system (CNS) are very sensitive to compression and could otherwise change the regenerative response of the surrounding environment. The diffusion coefficient, however, was of the order of the one of small gas molecules diffusing through a solid membrane.

### Brief description of the figures

Figure 1 shows the pore size distribution (µm) and porosity (fraction of the total pore volume, %) of the different layers of the tubular scaffold.
Figure 2 shows the diffusion of glucose through the hybrid duct (small molecule)
Figure 3 shows the diffusion of bovine serum albumin (BSA, larger molecule) through the tubular duct.
Figure 4 shows, through a confocal microscopy image, the results of diffusion through Schwann cells cultured 10 days inside the duct, whose cytoskeleton is marked in gray falcidin. The dashed line shows the limits of the channel: Cells can not pass through it.
Figure 5 is a scanning electron microscopy image of the same Schwann cell culture as in Figure 4, showing the channel with adhered cells, and the longitudinal section structure of the tube. No cells are detected either in the exterior or in the middle layer of the duct.

### Examples

### 1. Preparation of materials

A thin block of polytetrafluoroethylene (PTFE) with perforated grooves 1.5 mm wide was used as the mold for the ducts. A poly-ε-caprolactone (PCL, PolySciences) fiber of 400-450 µm in diameter was provided in each groove using PTFE washers with a 1.5 mm outer diameter every 3 cm of fiber to keep it centered. These fibers acted as a negative for the lumen of the ducts. HA solutions (Sigma-Aldrich) at 1,3 and 5 wt% HA, were prepared in a sodium hydroxide solution (NaOH, Scharlab) and were gently stirred. Divinyl sulfone was used as a crosslinking agent (DVS, Sigma-Aldrich) (by a 1,4 Michael addition) in a molar ratio of DVS: HA, monomer units, of 9:10. After addition, the solutions were stirred for additional 10 seconds and were injected into the grooves of the mold. Once the solution was gelled, the mold was placed in a Petri dish to avoid evaporation and was cooled to -20 °C. The mold-solution assembly was then lyophilized (Lyoquest-85, Telstar) for 24 h at 20 Pa and -80 °C to generate microporous HA matrices due to water sublimation. The fiber duct was then carefully withdrawn from the mold and the PTFE rings were removed. In order to extract the PCL fiber from each of the HA ducts, said fiber was stretched from its ends to reduce its diameter. Finally, the ducts were cut into 6 mm portions and stored at 4-8 ° C in 30 sterile distilled water until use (up to 4 weeks).

HA ducts were obtained after lyophilization of HA solutions at 1,3 and 5 wt % of HA, injected into the molds together with the cross-linking agent. The result was a soft, stable duct with dimensions of 5.384 ± 0.246 mm in length and 1.251 ± 0.117 mm in width. This duct had a centered inner channel of 0.406 ± 0.056 mm in diameter. The central channel was sufficiently wide under wet conditions to allow insertion of PLLA fibers in its interior.

The central channel extends from one end to the other of the scaffold. In the case of HA-PLLA the soft fibers are arranged parallel to the surface of the channel to favor the extension of the cells on them.

A structural study using scanning electron microscopy (SEM) images of the porosity in different zones of the wall of HA ducts at 5% by weight, revealed a unique permeable substrate, in which three pore topologies were observed. The surface of the channel had a continuous and homogeneous layer with micropores; the internal structure showed larger interconnected honeycomb-like pores, and the outer surface was rough with a random cavities distribution.

### 2. Cells and Hybrid Duct

Primary cultures of Schwann rat cells (SCs, Innoprot) were used. SCs were grown in flasks and were grown to converge at 37 °C, 5% CO2, in a complete medium containing essential and non-essential amino acids, vitamins, organic and inorganic compounds, hormones, growth factors, trace minerals and 10% of fetal bovine serum (P60123, Innoprot). All experiments were performed with cells in passage 4 to 6. 5% HA ducts were disinfected with their hollow lumen or occupied by PLLA fibers, and their films were disinfected and preconditioned for cell culture experiments in an enclosure of laminar flow by means of two successive rinses with 70° ethanol for 1 hour. The samples were rinsed with ethanol at 50° and 30° for 10 minutes at a time, and then rinsed thoroughly with deionized water. The viability and proliferation of SCs were evaluated by the MTS assay (CellTiter 96 Aqueous One Solution, Promega). In HA ducts and 5 HA ducts with PLLA fibers in their lumen there was a significant increase in absorbance with the culture time, with respect to two-dimensional materials. The results obtained in both three-dimensional structures were of the same order for each culture and similar to those found for PLLA films on day 10. Viable and dead cells were stained and photographed by fluorescence microscopy; The images after 5 and 10 days of culture show a considerable amount of calcein-stained live cells for the three structures: HA ducts, HA-PLLA ducts and PLLA bundles. Cell mortality was greater on PLLA fibers than inside HA ducts, with or without such fibers. Quantitatively, flow cytometry analysis revealed a decrease in the percentage of dead cells inside the ducts with the time of culture (LIVE / DEAD Cell Viability Assay, Life Technologies), whereas this decrease of dead cells did not occur when the cells were cultured with the control (well plate culture well). The study of cell distribution inside the ducts by immunohistochemistry and the image processing revealed a uniform cell population after 10 days of culture along one end of the lumen, irrespective of whether or not it was filled with fibers. In those ducts containing PLLA fibers, the cells appeared to be better distributed along the lumen section, while the cells were rather wound up as a leaf in the empty lumen; this fact is reflected as the deviation of the mean intensity along the ducts, which is greater in the first case. Finally, the identity of the cells was confirmed as SCs by staining of anti-GFAP, anti-p75 and anti-S100 antibodies, and their morphology was revealed by falcidin in ducts with or without fibers. In HA ducts SCs achieved a high degree of confluence after 10 days of culture and had cellular processes, often branched. The cells spread and proliferated as a layer and migrated along the lumen. However, on the PLLA fibers the SCs cells were aligned with respect to the long axis of the fibers and showed a bipolar morphology, with a mainly spindle shape and established cell-cell contacts. In multiphoton imaging it was possible to observe, without the need of any cut, that the cells were accommodated coating the lumen of the HA and HA-PLLA ducts. Similar results could be confirmed by scan electron microscopy (SEM) images, in which the details allow to assess the different degree of adhesion of the cells depending on the substrate: the cells showed a round conformation forming aggregates and establishing adhesions located on the surface of the HA lumen, but elongated and completely adhered to the fibers, revealing intimate PLLA-cells contact. Expression of myelin glycoprotein (p0) after 10 days of culture increased compared to 1 day in HA and HA-PLLA ducts. The expression of myelin zero protein (p0), which encodes the major myelin protein (constituting more than 50% of the total protein in mature Schwann cells) and is involved in the adhesion of membranes in spiral wrappings of myelin sheaths, in processes of compaction, interestingly increased in a 3D environment without addition of any axonal signal. This expression p0 is barely detectable after 1 day, but its presence is massive in the ducts after 10 days, both with fibers and without fibers.

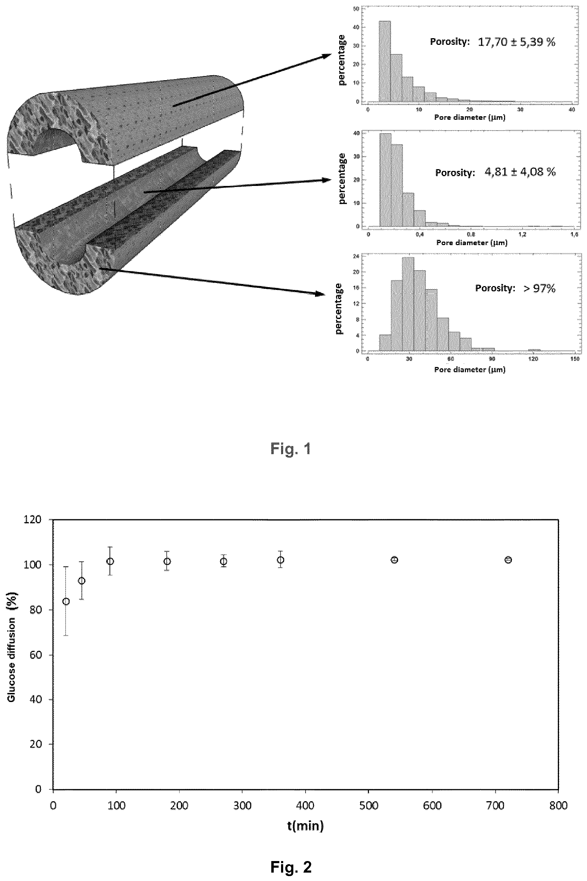

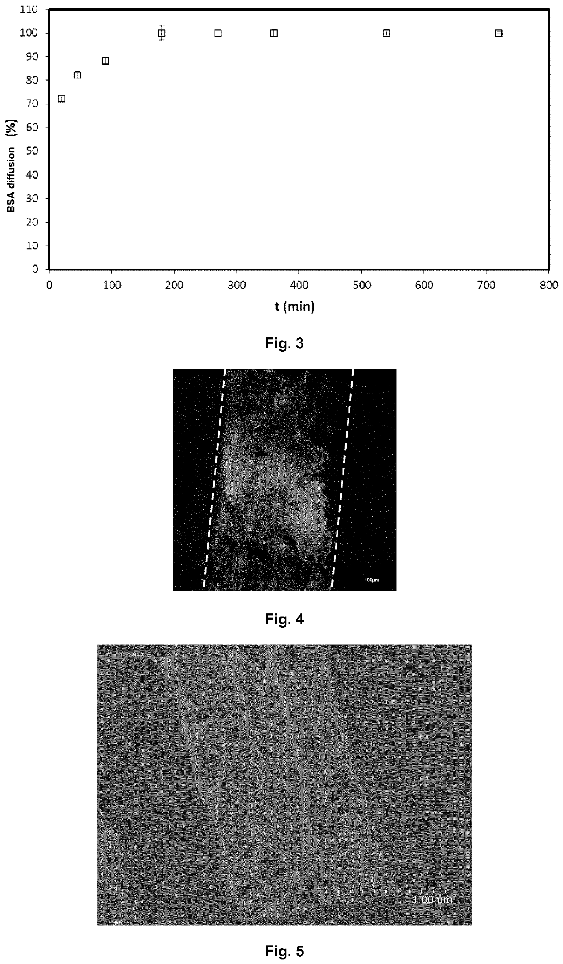

Figures 2 and 3 show that both small molecules of physiological interest, such as glucose, as proteins (such as BSA), can diffuse easily through the walls of the tube. Figures 4 and 5 show the effectiveness of channel confinement in cells that were seeded in the interior. This shows, at the same time, that cells from the outside cannot penetrate the channel. This property protects the cells inside the tube from possible aggressions from the environment.

As evidence that the three-layer membrane is not an obstacle to the passage of bioactive nutrients and molecules, but prevents migration of cells from the inside out, or penetration from outside, diffusion results are also presented through the duct of Schwann cells culture, Figures 4 and 5.

## Claims

1. A degradable implantable and biocompatible tubular scaffold **characterized in that** it comprises three layers of different porosity: an inner layer a), an intermediate layer b) and an outer layer c), with uninterrupted connection among them, and the three composed by a same porous hydrogel based on crosslinked hyaluronic acid.

2. Tubular scaffold according to claim 1, **characterized in that** the porous hydrogel layers have a porosity:
- the inner layer a) has micropores of less than 1 µm;
- the intermediate layer b) has interconnected, honeycomb-like pores, larger than those of the inner and outer layers, of size between 10 to 70 µm and
- the outer layer c) has irregular pores smaller than 12 µm in size.

3. Tubular scaffold according to any one of the preceding claims, **characterized in that** it has an internal diameter with dimensions of about 400 µm and a length of up to 50 mm.

4. A bio-hybrid **characterized in that** it comprises a tubular scaffold defined in any one of the preceding claims.

5. Biohybrid according to claim 4, **characterized in that** it comprises Schwann cells or olfactory envelope glia in its interior.

6. Biohybrid according to one of claims 4 or 5, **characterized in that** it further comprises growth factors and / or drugs in its lumen.

7. Biohybrid according to claim 6, **characterized in that** the growth factors are selected from neurotrophins NGF, BDNF or GDNF.

8. Biohybrid according to claim 6, **characterized in that** the drugs are dopaminergics.

9. Biohybrid according to claim 6, **characterized in that** growth factors and / or drugs are present in the lumen embedded in gels or microparticles.

10. Biohybrid according to claim 9, **characterized in that** the gels are injectable and *in situ* gelifiable peptides or solutions of hydrogels, preferably fibrin, collagen or agarose.

11. Biohybrid according to claim 9, **characterized in that** the microparticles have a hydrophilic character.

12. Biohybrid according to claim 9, **characterized in that** the microparticles have hydrophobic character.

13. Biohybrid according to claim 9, **characterized in that** the microparticles are of PLLA or cross-linked gelatin.

14. Biohybrid according to one of claims 4 to 13, **characterized in that** it comprises microfilaments of degradable synthetic polyesters of nylon or silk of diameters from microns to tens of microns, arranged in parallel in the lumen, which serve as support for the adhesion and guidance to the migration of cells and the extension of axons. 35

15. Method for obtaining the tubular scaffold defined in one of claims 1 to 3, **characterized in that** it comprises:
- providing a grooved mold for containing said tubular scaffold,
- introducing into said mold a polymer material in the form of fiber(s),
- preparing HA solutions and stirring them in the presence of a cross-linking agent,
- injecting said solutions into the grooves of the mold, obtaining a mold-solutions assembly which cross-links *in situ,*
- freezing the mold-solution assembly obtained, and
- lyophilizing the mold-solution assembly obtaining microporous HA matrices.

16. Method according to claim 15, **characterized in that**:
- the mold is of a hydrophobic polymeric material
- the polymer material in the form of fibers is of a hydrophobic polymeric material
- the cross-linking agent is divinyl sulfone, glutaraldehyde or carbodiimide.

17. Method according to claim 15, **characterized in that**:
- the mold is of polytetrafluoroethylene
- the polymeric material in the form of fibers is of poly-ε-caprolactone 5
- the cross-linking agent is divinyl sulfone, glutaraldehyde or carbodiimide.

18. Method according to one of claims 15 to 17, **characterized in that** it comprises after the lyophilization step:
- withdrawing the tubular scaffold from the mold and the rings of material forming the mold itself
- removing the fiber of polymeric material, obtaining a duct with a centered inner channel, and
- hydrating the HA ducts.

19. Method according to one of claims 15 to 18, **characterized in that** it comprises after the hydration step, the insertion of PLLA fibers in its interior.

20. A tubular scaffold defined in one of claims 1 to 3, **characterized in that** it is obtained by a method as defined in one of claims 15 to 19.

21. A tubular scaffold defined in one of claims 1 to 3 or a biohybrid defined in one of claims 4 to 14 for use in inducing the regeneration of neural tracts and the reconnection of damaged or degenerate neuronal populations.

22. A tubular scaffold defined in one of claims 1 to 3 or a biohybrid defined in one of claims 4 to 14 for use in the regeneration of tracts in diseases affecting the central nervous system, preferably Parkinson's disease and spinal cord injuries.
